# EUROPÄISCHE PATENTSCHRIFT

(11) **EP 0 966 684 B1**
(45) Veröffentlichungstag und Bekanntmachung des Hinweises auf die Patenterteilung: **25.02.2004**
(21) Anmeldenummer: 98917057.6
(22) Anmeldetag: 23.03.1998
(51) Int. Cl.: G01N 33/52, G01N 33/68, C12Q 1/48, C07D 311/90

(54) **VERBINDUNGEN ZUM NACHWEIS VON PHOSPHORSÄUREESTERN**
COMPOUNDS FOR DETECTING PHOSPHORIC ACID ESTERS
COMPOSES POUR L'IDENTIFICATION DES ESTERS D'ACIDE PHOSPHORIQUE

(30) Priorität: 21.03.1997 DE 19711796
(43) Veröffentlichungstag der Anmeldung: 29.12.1999
(73) Patentinhaber: Gesellschaft für Biotechnologische Forschung mbH (GBF), 38124 Braunschweig (DE)
(72) Erfinder: TEGGE, Werner, D-38124 Braunschweig (DE); GAST, Rainer, D-38124 Braunschweig (DE); GLÖKLER, Jörn, D-38124 Braunschweig (DE)
(74) Vertreter: Boeters, Hans Dietrich, Dr.
(86) Internationale Anmeldenummer: PCT/EP1998/001689
(87) Internationale Veröffentlichungsnummer: WO 1998/043082

(56) Entgegenhaltungen:
- EP-A- 0 178 775
- EP-A- 0 314 480
- WO-A-95/27796
- US-A- 5 576 433

## Beschreibung

Die vorliegende Erfindung betrifft die Verwendung von Verbindungen (Marker) zum Nachweis von Phosphorsäureestern, wobei die Verbindungen einen Chelatbildner, ein Zentralatom und einen Fluoreszenzfarbstoff umfassen.

Phosphorsäureester sind lebenswichtige Komponenten biologischer Systeme und Bestandteile einer großen Zahl von biochemischen Verbindungen. Sie spielen insbesondere bei der Funktion und Regulation von zellulären Prozessen eine wichtige Rolle, wo sie z.B. als Zwischenprodukte in zahlreichen Stoffwechselprozessen vorkommen. Biologisch wichtige Phosphorsäureester sind z.B. Adenosinmono-, di- und -triphosphate, Lecithine, Kephaline, Phospholipide, Nucleotide und Coenzyme. Eine besonders große Rolle bei Regulationsvorgängen spielt auch die Phosphorylierung und Dephosphorylierung von Proteinen durch Proteinkinasen bzw. Phosphatasen. Der Nachweis von phosphorylierten Molekülen bzw. die Analyse des Phosphorylierungszustandes von Biomolekülen ist daher von großer Bedeutung in der biologischen Forschung.

Es ist bereits bekannt, phosphorylierte Verbindungen mit 32_{P} oder 33_{P} radioaktiv zu markieren und die entsprechenden Radionuklide nachzuweisen. Derartige Verfahren weisen jedoch den Nachteil auf, daß sie nur unter großen Sicherheitsvorkehrungen durchgeführt werden dürfen.

Es ist weiter bekannt, spezifische Antikörper zum Nachweis von phosphorylierten Verbindungen einzusetzen. Die Herstellung von Antikörpern ist jedoch aufwendig und damit teuer und ihre Spezifität wird stark von Strukturen außerhalb der zu analysierenden Phosphatester beeinflußt. Es besteht daher weiterhin ein Bedarf an alternativen Verbindungen zum Nachweis von Phosphorsäureestern.

EP-A-0 314 480, EP-A-0 178 775 und US-A-5 576 433 offenbaren lediglich Chromophore die an einen mehrzähnigen Chelatbildner gebunden sind zum Nachweis von Ca²⁺. EP-A-0 314 480 lehrt diese Verbindungen mit Xanthenfarbstoffen und Fluoreseein-Fluorophoren.

WO-A-9 527 796 offenbart die Trennung phosphorylierter Peptide von nicht-phosphorylierten Peptiden, die dadurch geschieht, daß erstere über Fe³⁺ Chelatbildner-Komplexe (N-Iminodiessigsäuregruppen) an eine Festphase gebunden werden. Die Peptidsubstrate sind dabei mit einem Farbstoff markiert, so daß die Menge Festphasen-gebundenen phosphorylierten Peptides bestimmt werden kann.

Es ist somit die Aufgabe der vorliegenden Erfindung, Verwendungen von Verbindungen bereitzustellen, die für einen sehr empfindlichen, ungefährlichen und spezifischen Assay, wie z.B. den Nachweis von Phosphorsäureestern, oder die Trennung von Verbindungen wie Proteinen oder Peptiden einsetzbar sind, wobei keine Radionuklide oder Antikörper zum Einsatz kommen müssen.

Diese Aufgabe wird gelöst durch die Verwendung einer Verbindung, die
a) mindestens einen Chelatbildner,
b) mindestens ein mit dem (den) Chelatbildner(n) koordiniertes zentralatom bzw. -ion, und
c) mindestens einen an den (die) Chelatbildner gebundenen Farbstoff umfaßt, zur Markierung von Phosphorsäureestergruppen enthaltenden Peptiden oder Proteinen.

Bevorzugte Ausführungsformen sind Gegenstand der abhängigen Ansprüche.

Dabei werden bevorzugt mehrzähnige Chelatbildner wie Iminodiessigsäure (IDA) und deren Derivate, Nitrilotriessigsäure und deren Derivate, Polyoxycarbonsäuren und deren Derivate, Polyamine und deren Derivate oder Ethylendiaminessigsäure und deren Derivate eingesetzt.

Die selektive Bindung von IDA über eine Fe³⁺- Chelatisierung an Phosphatester ist bereits bekannt, vgl. Anderegg, G. & Schwarzenbach, G. (1955) Helv. Chim. Acta 38, 1940-1942; Muszynska, G. & al. (1992) J. Chromat. 604, 19-28; Schwarzenbach, G. & al. (1955) Helv. Chim. Acta 38, 1147-1170; Songyang, Z., Blechner, S., Hoagland, N., Hoekstra, M.F., Piwnica-Worms, H. & Cantley, L.C. (1994) Current Biology 4, 973-981. Es ist auch bekannt, immobilisierte IDA für Affinitätsreinigungen bzw. Anreicherungen von Biomolekülen an Chromatographieträgern und Membranen einzusetzen: So wird von der Firma Pharmacia Biotech ein entsprechendes chelatisierendes Chromatographiematerial unter dem Namen Chelating Superose/Sepharose vertrieben.

Ferner wird von der Firma QIAGEN ein Protein-Reinigungssystem vertrieben. Das System kann zur Isolierung von Proteinen und Peptiden eingesetzt werden, die mit sechs aufeinanderfolgenden Histidinresten markiert wurden. Diese Reste weisen eine hohe Affinität für immobilisierte Nickelionen auf, die ihrerseits durch Chelatisierung an Nitrilotriessigsäure-Harze (Ni-NTA) gebunden sind. Ein wesentlicher Nachteil dieses Systems besteht jedoch darin, daß die Proteine und Peptide mit den Histidinresten markiert werden müssen.

Weiter werden von der Firma QIAGEN Ni-NTA-Konjugate mit Antikörpern, Peroxidase, alkalischer Phosphatase und Meerettichperoxidase angeboten.

Der Einsatz des Chelatisierungseffekts für Assays, insbesondere für den direkten Nachweis und/oder die Trennung von Phosphatestern durch eine Verbindung eines Chelatbildners mit einem Farbstoff (Chromophor) ist jedoch noch nicht bekannt.

Erfindungsgemäß ist das mit dem Chelatbildner koordinierte mindestens eine Zentralatom bzw. -ion vorzugsweise ein Metallatom bzw. - ion, insbesondere ein Übergangsmetallatom bzw. -ion wie Eisen und/oder Nickel, kann aber auch eine (oder mehrere) andere Gruppierung (en) darstellen, die einsame Elektronenpaare oder Elektronenlücken aufweisen. Bevorzugt wird Fe³⁺ eingesetzt.

Kommen mehrere Zentralatome bzw. -ionen zum Einsatz so können die erfindungsgemäß zu verwendenden Verbindungen einen oder mehrere Brückenliganden umfassen.

Die erfindungsgemäß eingesetzten Farbstoffe sind vorzugsweise chemisch an den Chelatbildner gebunden, können aber auch auf andere Weise mit dem Chelatbildner assoziiert sein. Der hier verwendete Begriff Farbstoffe umfaßt neben üblichen Farbstoffen auch Pigmente und andere chromophore Gruppen und deren Edukte, die z.B. erst nach der Umsetzung mit dem Chelatbildner(n) zu Chromophoren werden. Als Farbstoffe können z.B. Xanthenfarbstoffe wie Fluoreszenzfarbstoffe, insbesondere Fluorescein und dessen Salze und Derivate wie Fluoresceinisothiocyanat zum Einsatz kommen.

Eine bevorzugte erfindungsgemäß zu verwendende Verbindung ist der Eisen(III)-Komplex von N-Iminodiessigsäureethyl-N'-fluoresceinylthioharnstoff.

Die Verbindungen können durch ein Verfahren hergestellt werden, bei dem mindestens einer der vorstehend aufgeführten Chelatbildner mit mindestens einem der vorstehend bezeichneten Farbstoffe unter geeigneten Bedingungen umgesetzt wird und dann mindestens eines der vorstehend definierten Zentralatome bzw. -ionen unter geeigneten Bedingungen zugesetzt wird.

In dem folgenden Schema wird eine bevorzugte Ausführungsform des Verfahrens dargestellt: Eine Aminoethyliminodiessigsäure (1) wird mit Fluoresceinisothiocyanat (2) zur Verbindung (3) umgesetzt. Nach Behandlung mit FeCl₃ kann das Chelat (4) ausgefällt werden.

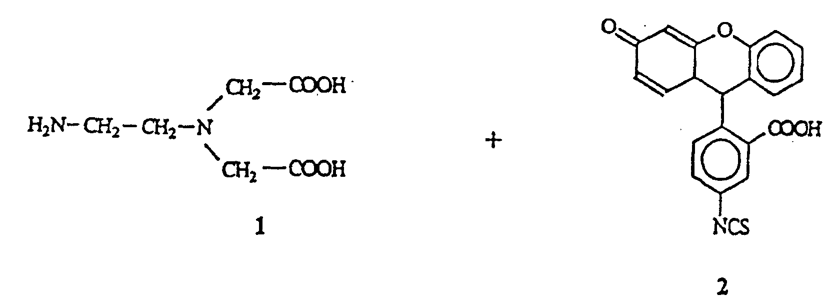

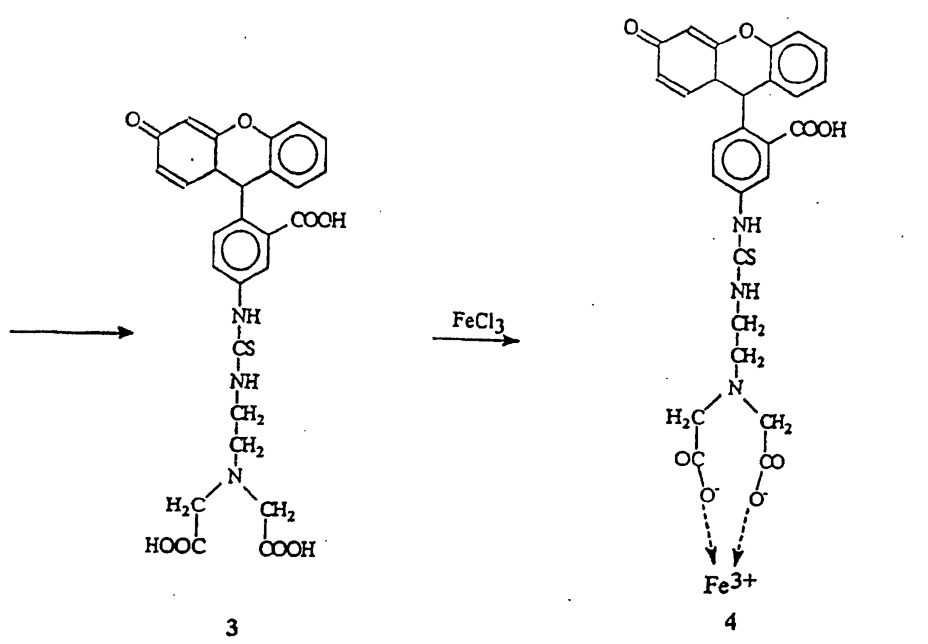

Die erfindungsgemäβ zu verwendenden Verbindungen können in einem Assay, bevorzugt zum Nachweis und/oder zur Trennung von zumindest teilweise Phosphorsäureestergruppen enthaltenden Verbindungen verwendet werden. Dabei sind folgende Variationen möglich:

Es können Proteine oder Peptide analysiert und getrennt (auch physikalisch getrennt) werden, die gleiche oder nahezu gleiche Sequenzen aufweisen, wobei die Peptide oder Proteine oder jeweilige Gruppen davon eine unterschiedliche Anzahl von Phosphorsäureestergruppen aufweisen. Der Ausdruck "nahezu gleiche Sequenzen" bedeutet dabei vorzugsweise, daß sich die Sequenzen um nicht mehr als eine Aminosäure pro 20 Aminosäuren, stärker bevorzugt pro 30 Aminosäuren, noch stärker bevorzugt pro 40 Aminosäuren und noch stärker bevorzugt pro 100 Aminosäuren der Gesamtfrequenz unterscheiden.

So können z.B. Proteine oder Peptide analysiert und/oder getrennt werden, die sich nur durch die Anzahl ihrer Phosphorsäureestergruppen unterscheiden. Dabei kann wie folgt vorgegangen werden:

Es wurden z.B. zwei Beadsorten hergestellt, die jeweils eine Octapeptidsequenz tragen und sich nur dadurch unterscheiden, daß die eine Sequenz ein Serinphosphat und die andere ein unmodifiziertes Serin enthält. Nach der Inkubation der Beads mit dem vorstehend genannten IDA Derivat (4) wurden die beiden Populationen durch Messungen in einem FACS-Gerät (Zell-Scanner und -Sorter) untersucht. Das Ergebnis wird in der folgenden Abbildung dargestellt, aus der hervorgeht, daß die Fluoreszenzmarkierung der Sequenz mit dem Serinphosphat deutlich stärker ist; die beiden Beadsorten sind annähernd Grundlinien-getrennt.

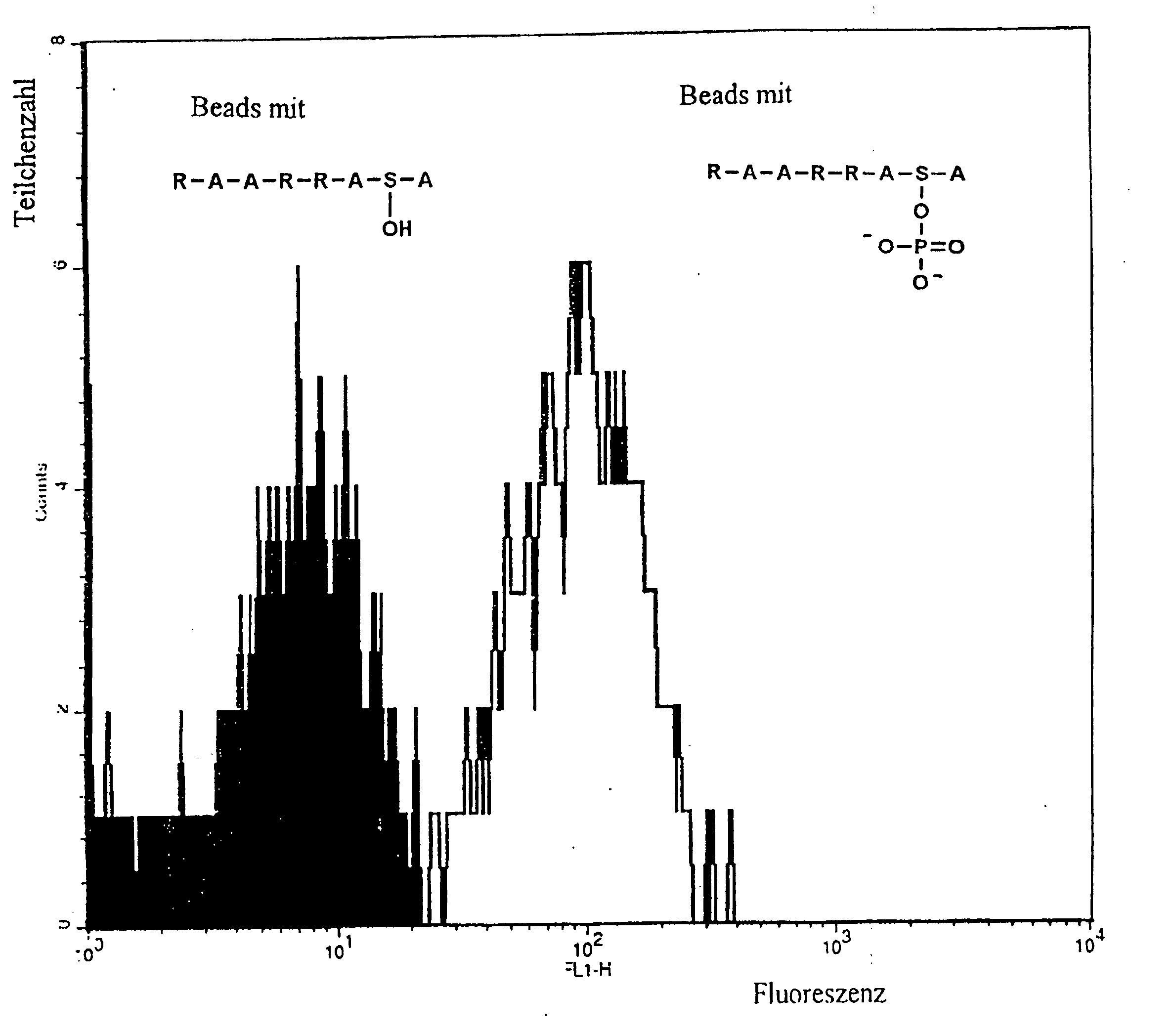
Teilchenzahl
8
Beads mit
R–A–A–R–R–A–S–A
|
OH
Beads mit
R–A–A–R–R–A–S–A
|
O
|
⁻O–P=O
|
O⁻
6
Counts
4
2
0
10⁰
10¹
10²
FL1-H
10³
10⁴
Fluoreszenz

Weiter können Proteine oder Peptide analysiert und/oder getrennt (auch physikalisch getrennt) werden, die sich sowohl bezüglich der Anzahl -ihrer Phosphorsäureestergruppen als auch bezüglich ihrer Sequenzen unterscheiden. Dabei kann wie folgt vorgegangen werden:

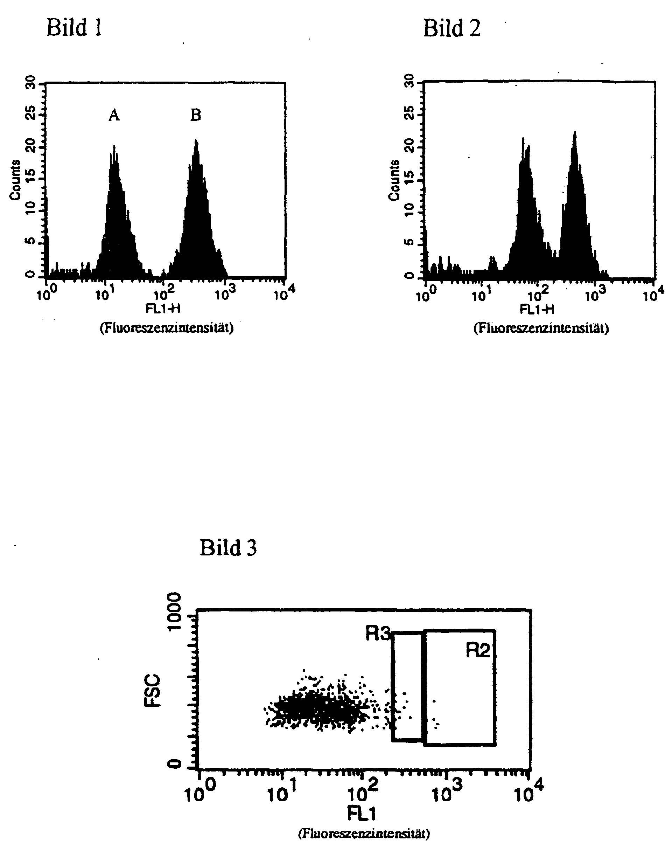

Zwei Sorten von Agarose-Beads (20-40 µm Durchmesser) mit den gebundenen Peptid-Heptasequenzen X-X-X-X-S-A-A (mit X = gleiche Anteile an Phenylalanin, Tyrosin und Tryptophan; S = Serin, unphosphoryliert), und A-A-R-R-S(P)-A-A (mit S (P) = phosphoryliertes Serin, nach Inkubation der Beads mit PKA) wurden nach ihrer 1:1 Mischung mit einer erfindungsgemäßen Verbindung behandelt und am FACS-Gerät untersucht. Bild 1 zeigt die beiden Beads-Populationen der phosphorylierten (B) und nicht-phosphorylierten (A) Sequenzen. Die Abszisse gibt die Fluoreszenz-Intensitäten an. Es ist eine klare Unterscheidung der beiden Populationen und eine physikalische Sortierung in Cell-Sortern möglich.

Weiter können Proteine oder Peptide analysiert und/oder aufgetrennt (auch physikalisch getrennt) werden, die eine gleiche oder ähnliche Anzahl von Phosphorsäureestergruppen, aber andere Unterschiede, wie unterschiedliche Peptid- oder Proteinsequenzen aufweisen. Dabei kann die physikalische Sortierung (Trennung) in Cell-Sortern stattfinden.

Der Ausdruck "eine ähnliche Anzahl von Phosphorsäureestergruppen" bedeutet vorzugsweise, daß die Anzahl der Phosphorsäureestergruppen der unterschiedlichen Peptide oder Proteine um maximal 10%, vorzugsweise um maximal 5%, noch stärker bevorzugt um maximal 2 % und am stärksten bevorzugt um weniger als 1%, bezogen auf die Anzahl der Phosphorsäureestergruppen der Verbindung mit den meisten Phsophorsäureesiergruppen, variiert, wobei der Unterschied mindestens eine Phosphorsäureestergruppe beträgt.

Der Ausdruck "unterschiedliche Sequenzen" bedeutet dabei vorzugsweise, daß sich die Sequenzen um mindestens 1 Aminosäure pro 19 Aminosäuren der Gesamtsequenz unterscheiden.

Dabei kann wie folgt vorgegangen werden:

Es werden Beads mit den entsprechenden unphosphorylierten Sequenzen (z. B. X-X-X-X-S-A-A und A-A-R-R-S-A-A) gemischt und mit PKA (Proteinkinase) inkubiert und phosphoryliert. Nach der Behandlung mit einer erfindungsgemäßen Verbindung wurde das Ergebnis gemäß Bild 2 erhalten. Es erfolgte eine klare und eindeutige Trennung der beiden Populationen, die eine physikalische Sortierung in Cell-Sortern erlaubt.

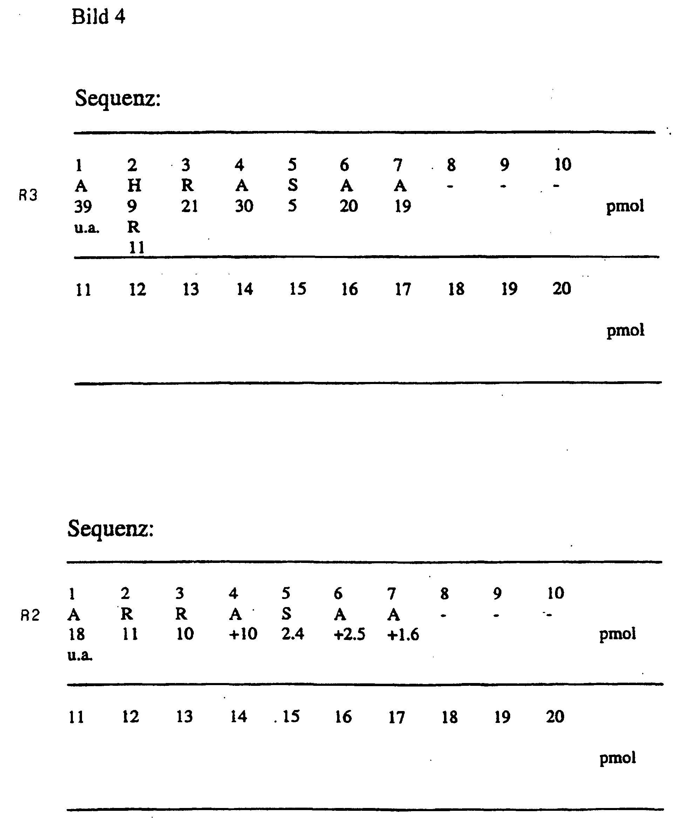

In einem weiteren Beispiel werden Beads mit der synthetisch dargestellten Peptid-Bibliothek A-X-X-A-S-A-A, (X= alle natürlichen Aminosäuren, exc. Cys) mit PKA inkubiert und anschließend mit einer erfindungsgemäßen Verbindung behandelt. Die Beads mit der höchsten Fluoreszenz wurden mit einem FACS-Sorter selektiert (zwei Populationen, R2 und R3, gemäß Bild 3). Die Pool-Sequenzanalyse der gesammelten Beads (Bild 4) zeigt, daß die Beads an den Positionen X hauptsächlich Arginin (R) tragen, was dem bekannten Erkennungsmotiv dieser Proteinkinase entspricht.

Das Verfahren ist also geeignet, für eine große Zahl von, z.B. identifizierten aber wenig charakterisierten, Proteinkinasen Konsensusmotive zu identifizieren.

Die Verbindungen lassen sich auch in vielen weiteren Assays einsetzen, wie z.B. bei der Analyse und Trennung von Phosphorsäureestergruppen enthaltenden Proteinen und Peptiden in Proteingelen bzw. auf Blots, bei der Kapillarelektrophorese und der Gelelektrophorese:

### Kapillarelektrophorese:

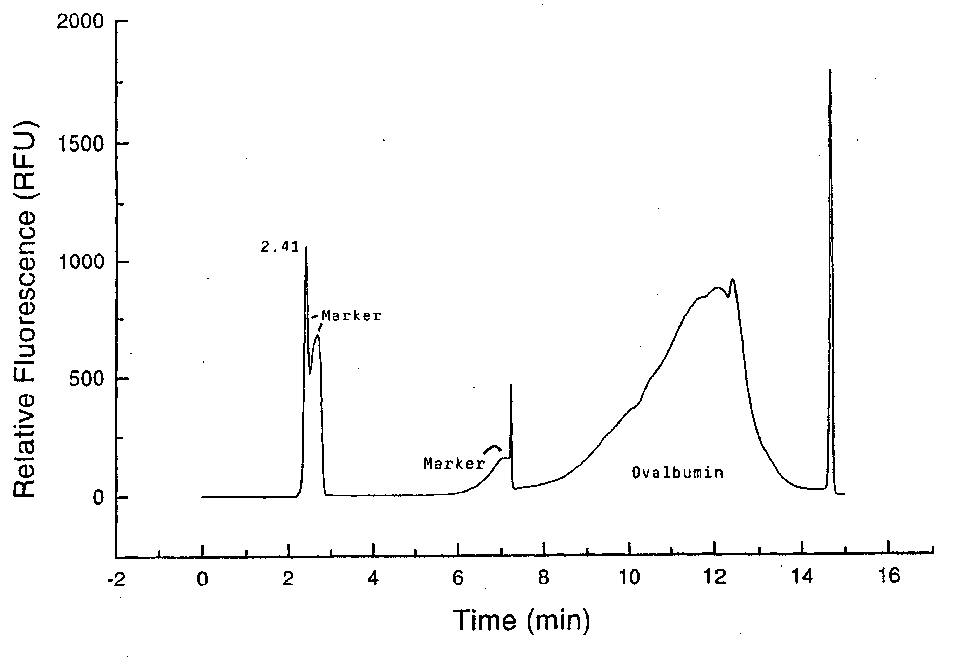

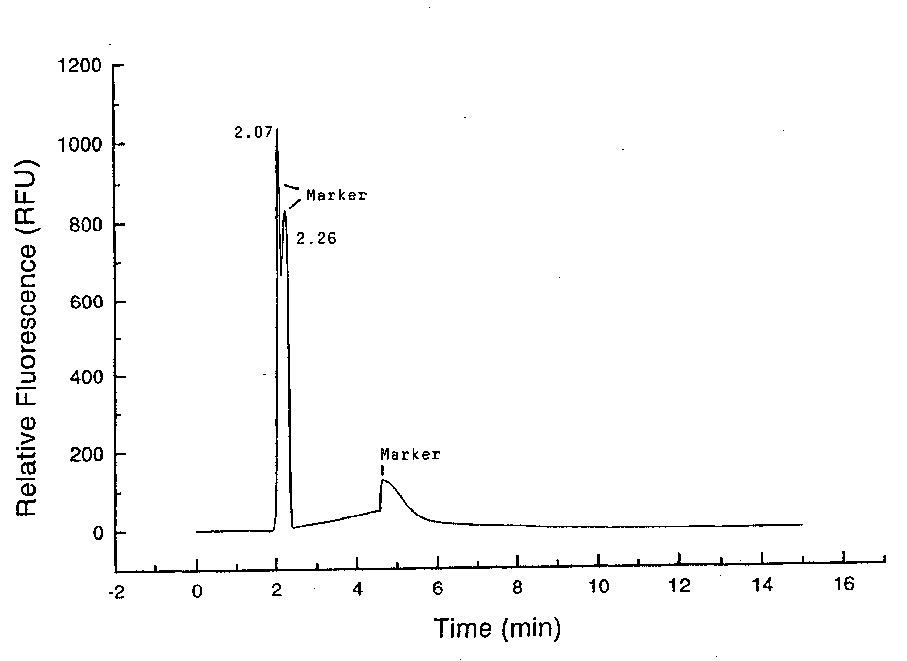

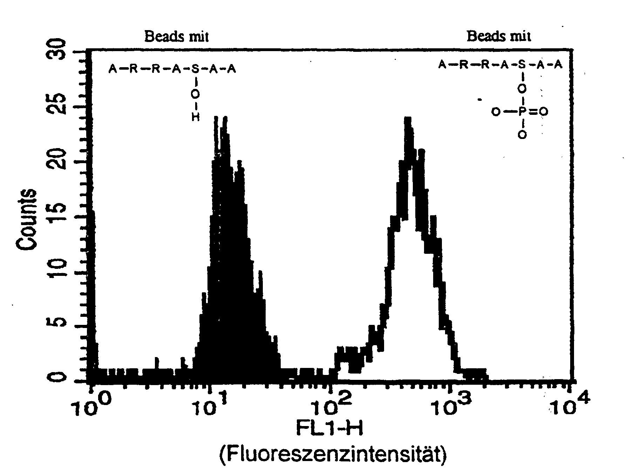

In Trennläufen auf einem Kapillarelektrophorese-System ließ sich Ovalbumin als phosphoryliertes Protein intensiv mit dem erfindungsgemäßen Fluorescein-IDA-Eisen Komplex markieren und mit einem entsprechenden Detektor nachweisen (Bild 5). Das unphosphorylierte Kontrollprotein Glucoce-Oxidase zeigte nur eine schwache Hintergrund-Fluoreszenz (Bild 6).

Dieser Einsatzbereich erlaubt die effiziente Kontrolle von biologischen Phosphorylierungsprozessen bei *in vitro* und *in vivo* Assays.

### Protein-Blots:

Proteine lassen sich durch Gelelektrophorese auftrennen. Die einzelnen Proteine können anschließend auf eine Trägermembran geblottet werden. Auf solchen Blots (Nitrozellulose) wurden phosphorylierte Proteine mit dem Fluorescein-IDA-Eisen Komplex spezifisch markiert.

Schließlich können die erfindungsgemäß zu verwendenden Verbindungen zum Nachweis von rekombinaten Peptiden oder Proteinen mit His-Tags (z. B. einer zusätzlichen Sequenz von 6 Histidinresten) eingesetzt werden, wobei als Zentralatom bzw. -ion andere Übergangsmetalle als Fe, wie z.B. Cu-, Ni-, Co- und/oder Zn-atome bzw. -ionen eingesetzt werden können.

Zusammenfassend ist festzustellen, daß die vorliegende Erfindung z. B. eine leichte Quantifizierung und Trennung von Proteinen und Peptiden ermöglicht, weil eine stöchiometrische, zeitlich stabile Quantifizierung der Fluoreszenzgruppe erfolgt. Ein weiterer Vorteil ergibt sich aus dem reversiblen Charakter der Färbung, die durch Zugabe von starken Chelatbildnern (EDTA), oder Veränderung des pHs rückgängig gemacht werden kann.

### Beispiele

### Darstellung von N-Carbethoxyethylendiamin

15 g (0,13 Mol) Diethylcarbonat werden mit 10 g (0,17 Mol) Ethylendiamin im Ölbad unter Rückflußkühlung für fünf Stunden auf 90°C erhitzt. Anschließend wird der bei der Umsetzung gebildete Ethylalkohol abrotiert. Das Produkt wird aus dem Rückstand in einer Kugelrohrdestillationsapparatur destilliert.
Übergang: 160°C (4 mbar)
Ausbeute: 8,5 g (50% d. Th)

### Darstellung von N-Carbethoxy-β-aminoethylimino-diessigsäure-dimethylester

8,5 g (64 mMol) N-Carbethoxyethylendiamin, 8,85 g (64 mMol) Kaliumcarbonat und 19,58 g (128 mMol) Bromessigsäuremethylester werden in dieser Reihenfolge zusammengegeben. Es tritt eine heftige Reaktion ein, nach deren Abklingen die Mischung vier Stunden im Ölbad unter Rückfluß auf 95°C erhitzt wird. Nach etwa einer halben Stunde setzt eine CO₂-Entwicklung ein, die kurz danach wieder abklingt. Nach Ablauf der Umsetzungszeit wird der Rückstand in 100 ml Diethylether ausgekocht, der Ether abrotiert und der Ester in einer Kugelrohrdestillationsapparatur destilliert.
Übergang: 220°C (0.1 mbar)
Ausbeute: 10,12 g (57% d. Th)

### Darstellung von Aminoethylimino-diessigsäure (IDA. 1)

10.12 g (37 mMol) N-Carbethoxy-ß-aminoethylimino-diessigsäure-dimethylester werden zu 650 ml einer 0.15 molaren Bariumhydroxid-Lösung gegeben. Die Mischung wird zwei Stunden im Ölbad unter Rückfluß auf 95°C erhitzt. Nach dem Abkühlen werden unter starkem Rühren 9,95 g einer 95-97%-igen Schwefelsäure zugetropft. Die Suspension wird anschließend zentrifugiert und einrotiert. Das Reinprodukt erhält man durch Umkristallisation aus Wasser/Ethanol. Ausbeute: 4.00 g (61% d. Th)

### Darstellung von N-Iminodiessigsäureethyl-N'-fluoresceinyl-thioharnstoff (3)

50 mg (0,28 mMol) Aminoethylimino-diessigsäure werden in 5 ml Wasser gelöst. Die Lösung wird anschließend mit einer 0.2 m Natriumhydroxid-Lösung auf pH 10.5 eingestellt. Hierzu pipettiert man 3 ml einer ethanolischen Fluoresceinisothiocyanat-Suspension (entsprechend 120 mg (0.30 mMol) FITC). Der pH sollte den Wert 8 nicht unterschreiten und wird im Wechsel mit der Zugabe der Suspension auf den Anfangswert nachkorrigiert.

Die pH-Korrektur erfolgt nach vollständiger Zugabe stündlich für noch etwa fünf Stunden. Die Mischung rührt dann 48 Std. unter Lichtausschluß bei Raumtemperatur und wird in dieser Zeit mehrmals auf pH 10.5 nachjustiert. Gegen Ende der Umsetzung bleibt der pH-Wert stabil. Die Lösung wird anschließend mit 1M HCl auf pH 2.5 eingestellt wobei das Produkt ausfällt. Dieses wird in 5 ml Ethanol gewaschen und im Vakuum getrocknet.
Ausbeute: 100 mg (66.6% d. Th)

### Darstellung des Eisen(III)-Komplexes des IDA-Derivats (4)

20 mg (0.035 mMol) N-Iminodiessigsäureethyl-N'-fluoresceinyl-thioharnstoff werden in 5 ml Wasser gelöst und die Lösung mittels Natronlauge auf pH 7,0 eingestellt. Der Lösung wird anschließend eine Lösung, bestehend aus 10 mg (0,037 mMol) FeCl₃ x 6 H₂O in 5 ml Wasser, zugesetzt, dabei fällt sofort ein schwarzer Feststoff aus, welcher abfiltriert und mit 5 ml Wasser nachgewaschen wird. Ausbeute: 20 mg

## Patentansprüche

1. Verwendung einer Verbindung, die
a) mindestens einen Chelatbildner,
b) mindestens ein mit dem (den) Chelatbildner(n) koordiniertes Zentralatom bzw. -ion, und
c) mindestens einen an den (die) Chelatbildner gebundenen Farbstoff umfaßt,
zur Markierung von Phosphorsäureestergruppen enthaltenden Peptiden oder Proteinen.

2. Verwendung nach Anspruch 1, **dadurch gekennzeichnet, daß** der (die) Chelatbildner aus mehrzähnigen Chelatbildnern wie Iminodiessigsäure und deren Derivaten, Nitrilotriessigsäure und deren Derivaten, Polyoxycarbonsäuren und deren Derivaten, Polyaminen und deren Derivaten und Ethylendiaminessigsäure und deren Derivaten ausgewählt wird (werden).

3. Verwendung nach einem der vorstehenden Ansprüche, **dadurch gekennzeichnet, daß** das (die) Zentralatom(e) bzw. -ion(en) mindestens ein Metallatom bzw. -ion, insbesondere mindestens ein Übergangsmetallatom bzw. -ion, speziell Fe³⁺ ist.

4. Verwendung nach einem der vorstehenden Ansprüche, **dadurch gekennzeichnet, daß** der (die) Farbstoff(e) mindestens ein Xanthenfarbstoff wie ein Fluoreszenzfarbstoff ist.

5. Verwendung nach Anspruch 4, **dadurch gekennzeichnet, daß** der Fluoreszenzfarbstoff einen Fluoresceinrest besitzt.

6. Verwendung nach Anspruch 1, nämlich des Eisen (III) -Komplexes von N-Iminodiessigsäureethyl-N'-fluoresceinylthioharnstoff.

7. Verwendung einer in einem der Ansprüche 1 bis 6 definierten Verbindung zur Identifikation von Konsensusmotiven von Proteinkinasen.

## Claims

1. Use of a compound that comprises
a) at least one chelating agent,
b) at least one central atom or central ion coordinated by the chelating agent(s), and
c) at least one dye bonded to the chelating agent(s) to mark peptides or proteins containing phosphoric ester groups.

2. Use according to claim 1, **characterised in that** the chelating agent(s) is (are) selected from multidentate chelating agents, such as iminodiacetic acid and derivatives thereof, nitrilotriacetic acid and derivatives thereof, polyoxycarboxylic acids and derivatives thereof, polyamines and derivatives thereof, and ethylenediamineacetic acid and derivatives thereof.

3. Use according to any one of the preceding claims, **characterised in that** the central atoms(s) or ion(s) is (are) at least one metal atom or ion, especially at least one transition metal atom or ion, expecially Fe³⁺.

4. Use according to any one of the preceding claims, **characterised in that** the dye(s) is(are) at least one xanthene dye, such as a fluorescent dye.

5. Use according to claim 4, **characterised in that** the fluorescent dye has a fluorescein residue.

6. Use according to claim 1, that is the iron (III) complex of N-iminodiacetic acid ethyl N'-fluoresceinyl thiourea.

7. Use of a compound **characterised in** any one of claims 1 to 6 for identifying consensus motifs in proteinkinases.

## Revendications

1. Emploi d'un composé qui comprend :
a) au moins un chélatant,
b) au moins un atome ou ion central coordiné avec le ou les chélatant(s),
c) et au moins un colorant lié au ou aux chélatant(s),
pour le marquage de peptides ou de protéines contenant des groupes esters phosphates.

2. Emploi conforme à la revendication 1, **caractérisé en ce que** le ou les chélatant(s) est ou sont choisi(s) parmi les chélatants polydentates tels que l'acide iminodiacétique et ses dérivés, l'acide nitrilotriacétique et ses dérivés, les acides polyoxycarboxyliques et leurs dérivés, les polyamines et leurs dérivés, et l'acide éthylènediaminetétraacétique et ses dérivés.

3. Emploi conforme à l'une des revendications précédentes, **caractérisé en ce que** le ou les atome(s) ou ion(s) central ou centraux est ou sont au moins un atome ou ion métallique, en particulier au moins un atome ou ion d'un métal de transition, et spécialement Fe³⁺.

4. Emploi conforme à l'une des revendications précédentes, **caractérisé en ce que** le ou les colorant(s) est ou sont au moins un colorant xanthénique comme un colorant fluorescent.

5. Emploi conforme à la revendication 4, **caractérisé en ce que** le colorant fluorescent comporte un reste de fluorescéine.

6. Emploi, conforme à la revendication 1, du complexe de fer Fe^{III} et de N-[bis(carboxyméthyl)aminoéthyl]-N'-fluorescéinyl-thiourée.

7. Emploi d'un composé défini dans l'une des revendications 1 à 6 pour l'identification de motifs consensus de protéine-kinases.
